# EUROPEAN PATENT SPECIFICATION

(11) **EP 3 719 132 B1**
(45) Date of publication and mention of the grant of the patent: **04.06.2025**
(21) Application number: 19784808.8
(22) Date of filing: 04.04.2019
(51) Int. Cl.: C12N 15/82, C12N 9/22, A01H 5/00, A01H 6/82, C12Q 1/6895

(54) **METHOD FOR OVERCOMING SELF-INCOMPATIBILITY OF DIPLOID POTATOES**
VERFAHREN ZUR ÜBERWINDUNG DER SELBSTINKOMPATIBILITÄT VON DIPLOIDEN KARTOFFELN
MÉTHODE POUR SURMONTER L'AUTO-INCOMPATIBILITÉ DE POMMES DE TERRE DIPLOÏDES

(30) Priority: 08.04.2018 CN 201810308517; 18.01.2019 CN 201910108556
(43) Date of publication of application: 07.10.2020
(73) Proprietor: Agricultural Genomics Institute, Chinese Academy of Agricultural Sciences, Shenzhen, Guangdong 518124 (CN); Agricultural Genomics Institute at Shenzhen, Chinese Academy of Agricultural Sciences, Shenzhen, Guangdong 518124 (CN)
(72) Inventor: HUANG, Sanwen, Shenzhen, Guangdong 518124 (CN); ZHANG, Chunzhi, Shenzhen, Guangdong 518124 (CN); PENG, Zhen, Shenzhen, Guangdong 518124 (CN); YE, Mingwang, Shenzhen, Guangdong 518124 (CN)
(74) Representative: Murgitroyd & Company
(86) International application number: PCT/CN2019/081515
(87) International publication number: WO 2019/196738

(56) References cited:
- EP-A1- 3 718 396
- WO-A1-2019/237808
- WO-A2-2020/018528
- CN-A- 105 052 724
- CN-A- 105 052 724
- CN-A- 106 939 316
- CN-A- 108 849 471
- CN-A- 109 548 646
- ENCISO ET AL: "Breaking Self-Incompatibility in Diploid Potato using the CRISPR/Cas9 System", PLANT AND ANIMAL GENOME XXVI CONFERENCE, 1 January 2018 (2018-01-01), XP055686267, Retrieved from the Internet <URL:https://pag.confex.com/pag/xxvi/meetingapp.cgi/Paper/27591> [retrieved on 20200416]
- ENCISO FELIX E.: "W988: Breaking Self-Incompatibility in Diploid Potato using the CRISPR/Cas9 System View Related Events", PLANT AND ANIMAL GENOME XXVI CONFERENCE, 13 January 2018 (2018-01-13), pages 1 - 1, XP055774505, Retrieved from the Internet <URL:https://pag.confex.com/pag/xxvi/meetingapp.cgi/Paper/30766> [retrieved on 20210210]
- ENCISO-RODRÍGUEZ FELIX ET AL: "Breaking self-incompatibility in diploid potato using genome editing", 16 January 2018 (2018-01-16), pages 1 - 3, XP055774512, Retrieved from the Internet <URL:https://pag.confex.com/pag/xxvi/recordingredirect.cgi/oid/recording3302/Paper30766_1.pdf> [retrieved on 20210210]
- YE MINGWANG ET AL: "Generation of self-compatible diploid potato by knockout of S-RNase", NATURE PLANTS, NATURE PUBLISHING GROUP UK, LONDON, vol. 4, no. 9, 13 August 2018 (2018-08-13), pages 651 - 654, XP036582670, DOI: 10.1038/S41477-018-0218-6
- FELIX ENCISO-RODRIGUEZ ET AL: "Overcoming Self-Incompatibility in Diploid Potato Using CRISPR-Cas9", FRONTIERS IN PLANT SCIENCE, vol. 10, 2 April 2019 (2019-04-02), XP055714526, DOI: 10.3389/fpls.2019.00376
- DZIDZIENYO DANIEL K ET AL: "Allelic diversity of S-RNase alleles in diploid potato species", THEORETICAL AND APPLIED GENETICS ; INTERNATIONAL JOURNAL OF PLANT BREEDING RESEARCH, SPRINGER, BERLIN, DE, vol. 129, no. 10, 6 August 2016 (2016-08-06), pages 1985 - 2001, XP036055967, ISSN: 0040-5752, [retrieved on 20160806], DOI: 10.1007/S00122-016-2754-7
- CAROLIN ANDERS ET AL: "Structural basis of PAM-dependent target DNA recognition by the Cas9 endonuclease", NATURE, vol. 513, no. 7519, 1 January 2014 (2014-01-01), London, pages 569 - 573, XP055240929, ISSN: 0028-0836, DOI: 10.1038/nature13579
- DZIDZIENYO DANIEL K ET AL: "Allelic diversity of S-RNase alleles in diploid potato species", THEORETICAL AND APPLIED GENETICS, vol. 129, no. 10, October 2016 (2016-10-01), pages 1985 - 2001, ISSN: 0040-5752(print)
- LUU, D.T. ET AL.: "S-RNase Uptake by Compatible Pollen Tubes in Gametophytic Self- incompatibility", NATURE, vol. 407, 5 October 2000 (2000-10-05), pages 649 - 651, XP055644707
- DZIDZIENYO, D.K. ET AL.: "Allelic Diversity of S-RNase Alleles in Diploid Potato Species", THEOR APPL GENET, vol. 129, no. 10, 6 August 2016 (2016-08-06), pages 1985 - 2001, XP036055967

## Description

### FIELD OF THE INVENTION

The invention relates to a CRISPR/Cas9 recombinant vector and use thereof as claimed, and a method for creating a self-compatibility potato as claimed. More specifically, the nucleotide sequence of the S-RNase protein targeted by the CRISPR/Cas9 recombinant vector is the sequence shown in SEQ ID NO:2 (Sp3), or a complementary sequence, a degenerate sequence, or a homologous sequence thereof; and/or the sequence shown in SEQ ID NO:3 (Sp4), or a complement sequence, a degenerate sequence, or a homologous sequence thereof.

### BACKGROUND OF THE INVENTION

A diploid potato belongs to gametophytic self-incompatibility type, and its pollen tubes can germinate on the stigma and elongate into the style, but the growth is subsequently inhibited. This trait is controlled by the S-RNase gene, and the expression of this gene inhibits the elongation of the pollen tubes, making it difficult for the potatoes to obtain an inbred plant line.

Previous studies have found that a tobacco S-RNase gene is only slightly expressed during the period from flower bud to bud stage, while higher S-RNase protein enrichment is detected in flowering stage. Our research on potato pistil proteins has also reaches the similar conclusion, and this creates the possibility of overcoming self-incompatibility of potatoes through self-crossing at the bud stage. However, self-crossing at the bud stage has higher requirements for pollination times and environment, lower fruit setting rate and fewer seeds, and the plants growing from the seeds of the self-crossing are still self-incompatible plants. The method is time-consuming and laborious, and the cost is extremely high; the self-compatibility is only exhibited in the generation for self-crossing at the bud stage, and is not heritable. The method of self-crossing at the bud stage cannot achieve the creation of self-compatible materials.

In 1998, Hosaka and Hanneman mapped a *Sli* (S-locus inhibitor) gene locus derived from the wild species *S. chacoense,* this gene confers self-compatibility to diploid potatoes. However, the infiltration of the *Sli* gene into a cultivar potato will inevitably bring in unfavorable traits, such as longer stolons, smaller potato tubers, and increased steroidal glycosides and alkaloids.

Therefore, it is desirable to create self-compatible potato materials in a better way. At present, no research institution has made breakthroughs in this regard.

The Plant and Animal Genome XXVI Conference reported "Breaking Self-Incompatibility in Diploid Potato using the CRISPR/Cas9 System" (Enciso ET AL, 1 January 2018, XP055686267). The Plant and Animal Genome XXVI Conference reported "W988: Breaking Self-Incompatibility in Diploid Potato using the CRISPR/Cas9 System View Related Events" (Enciso Felix E, 13 January 2018, pages 1-1, XP055774505). The Plant and Animal Genome XXVI Conference reported "Breaking self-incompatibility in diploid potato using genome editing" (Enciso-Rodriguez Felix ET AL, 16 January 2018, pages 1-3, XP055774512). The three reports were disclosed S-RNAse knockouts in Potato to confer self compatibility, and the knockouts are created using CRISPR/Cas9 gene editing.

Chinese patent application CN 105052724A describes a self compatible potato selection method based on detection of lower expression of S-RNAses.

The scientific publication entitled "Allelic diversity of S-RNase alleles in diploid potato species" (DZIDZIENYO DANIEL K ET AL, INTERNATIONAL JOURNAL OF PLANT BREEDING RESEARCH, vol. 1 29, no. 1 0, 6 August 201 6 (201 6-08-06), pages 1985-2001 , XP036055967) describes the S-ribonuclease sequences of 16 S-alleles derived from diploid potato. A phylogenetic analysis and partial phenotypic analysis supported the conclusion that these were functional S-alleles.

### BRIEF DESCRIPTION OF THE INVENTION

The invention is as defined in claims 1 to 3.

In order to solve the problem that there is no better method to create a self-compatible potato material in the prior art, the present invention provides a method for overcoming self-incompatibility of diploid potatoes. The purpose of the invention is to find a simple, accurate and efficient method to overcome self-incompatibility of diploid potatoes, i.e., overcoming the self-incompatibility barrier of diploid potatoes, thereby providing core technology support for the creation of a self-compatible material and a homozygous inbred plant line.

In order to achieve the above object, the present invention provides the following technical solutions

In a particular embodiment (not explicitly claimed) of the present invention, the gene regions of Sₚ₃ and Sₚ₄ in the S-RNase gene as a potato self-incompatibility determining gene comprise an exon and a promoter, or a portion thereof.

In a particular embodiment (not explicitly claimed) of the present invention, the homologous sequence of the nucleotide sequence of the S-RNase protein may be a polynucleotide hybridizing with a nucleotide sequence in SEQ ID NO:2 and/or SEQ ID NO:3 or a complementary sequence thereof under stringent conditions, or a fragment of the polynucleotide, wherein such a polynucleotide or a fragment thereof does not express the S-RNase protein.

In a particular embodiment (not explicitly claimed) of the present invention, the loss-of-function of the S-RNase gene is achieved by addition and/or deletion of (one or more) nucleotides in the gene expressing the S-RNase protein. For example, the addition of one, two or more nucleotides; or the deletion of one, two or more nucleotides; or the replacement of one, two or more nucleotides in the gene expressing the S-RNase protein.

Exemplarily (not explicitly claimed), the nucleotide sequence of the S-RNase protein is the sequence shown in SEQ ID NO:2 (Sₚ₃), or a complementary sequence, a degenerate sequence, or a homologous sequence thereof; and/or the sequence shown in SEQ ID NO:3 (Sₚ₄), or a complement sequence, a degenerate sequence, or a homologous sequence thereof.

In a particular embodiment (not explicitly claimed) of the invention, the addition, deletion or replacement of nucleotides is achieved by a CRISPR/Cas9 recombinant vector.

The present invention provides a CRISPR/Cas9 recombinant vector for targeted knockout of S-RNase genes, the nucleotide sequence of the S-RNase gene targeted by the CRISPR/Cas9 recombinant vector is the sequence shown in SEQ ID NO:2 (Sₚ₃), or a complementary sequence, a degenerate sequence, or a homologous sequence thereof; and/or the sequence shown in SEQ ID NO:3 (Sₚ₄), or a complement sequence, a degenerate sequence, or a homologous sequence thereof.

The nucleotide sequence in the CRISPR/Cas9 recombinant vector comprises:
S-RNase P3 (i.e., Seq ID No:4): xxxxACGATTCACGGGCTTTGGC,
S-RNase P4 (i.e., Seq ID No:5): xxxxGCCAAAGCCCGTGAATCGT;
wherein the portion not underlined is a sequence in above target site with deletion of NGG or a complementary sequence thereof, and the underlined portion is a cohesive end for ligation of the vector.

Disclosed herein is also the construction of the CRISPR/Cas9 recombinant vector comprises the following steps:
(1) designing primers S-RNase P3 and S-RNase P4 according to the target sequence;
(2) making S-RNase P3 and S-RNase P4 to form a double-stranded DNA having cohesive ends as an insert fragment for constructing the recombinant vector;
(3) digesting the pKSE401 vector with BsaI endonuclease as a skeleton fragment of the framework recombinant vector;
(4) ligating the recombinant vector backbone fragment and the insert fragment by T4 ligase, then transferring into *E. coli* to screen for the CRISPR/Cas9 recombinant vector.

Exemplarily, the nucleotide sequence in the CRISPR/Cas9 recombinant vector comprises:
S-RNase P3 (i.e., Seq ID No:4): xxxxACGATTCACGGGCTTTGGC,
S-RNase P4 (i.e., Seq ID No:5): xxxxGCCAAAGCCCGTGAATCGT;
wherein the portion not underlined is a sequence in above target site with deletion of NGG or a complementary sequence thereof, and the underlined portion is a cohesive end for ligation of the vector.

The invention provides use of the above CRISPR/Cas9 recombinant for knocking out an S-RNase protein gene of the diploid potato S.phureja.

Disclosed herein is also a method for creating a self-compatible potato. which comprises the step of making the S-RNase gene in a potato unexpressed or inactivated, wherein the S-RNase gene is the sequence shown in SEQ ID NO:2 (Sₚ₃), or a complementary sequence, a degenerate sequence, or a homologous sequence thereof; and/or the sequence shown in SEQ ID NO:3 (Sₚ₄), or a complement sequence, a degenerate sequence, or a homologous sequence thereof.

The method for creating a self-compatible potato specifically consists of:
(1) constructing a CRISPR/Cas9 recombinant vector;
(2) introducing by transformation the CRISPR/Cas9 recombinant vector in step (1) into potato cells, inducing the co-expression of the guide RNA expression cassette and the Cas9 nuclease expression cassette of the target fragment in the cell, cleaving the double-stranded target fragment of the S-RNase gene to trigger the DNA repair function of the potato cell itself, and causing random insertion or deletion of bases at the target site, thereby achieving a loss-of-function mutation of the intracellular S-RNase gene;
(3) screening for plants with a mutation in the S-RNase gene; and the potato is S.phureja.

The present invention adopts the above technical solutions, and it brings the following beneficial effects. Compared with the conventional methods for solving the self-incompatibility of potatoes, the present invention has the following advantages:
1) the invention performs directed editing of the self-incompatibility gene, constructing a vector which simultaneously targets the target sites of the two target genes, thereby creating a plurality of new self-compatible breeding materials;
2) the breeding period is short, and the entire process of the directed creation of the breeding material is about 12 months;
3) the artificial self-crossing is less affected by the flowering period and environment, saving time and labor, having strong maneuverability, higher fruit setting rate and more seeds, while self-crossing at the bud stage has large limitation, lower fruit setting rate and fewer seeds;
4) the self-compatibility of the self-compatible material created by the invention is heritable, and the barrier of self-incompatibility for diploid potatoes is fundamentally overcomed.

### BRIEF DESCRIPTION OF THE DRAWINGS

FIG. 1 shows the detection results of the target mutations according to an example of the present invention.
FIG. 2 shows the ploidy detection of the regenerated plants provided by an example of the present invention, wherein panel a is a diploid; panel b is a tetraploid.
FIG. 3 is a phenotype diagram showing elongation of pollen tubes in the style of a wild type material (A) and a genetically edited material (B) according to an example of the present invention.
FIG. 4 is a phenotype diagram showing the fruit setting for self-crossing of a wild-type and a genetically edited plant line according to an example of the present invention.

### DETAILED DESCRIPTION OF INVENTION

The invention will be further described in detail below with reference to a particular example and the drawings.

Example 1 is not encompassed by the wording of the claims but is considered as useful for understanding the invention.

**Example 1:** Example 1 provides a method for overcoming the self-incompatibility of diploid potatoes, which is useful for understanding the invention, and comprises the following steps:
(1) selecting a target fragment in the first exon region of Sₚ₃ and Sₚ₄ in the S-RNase gene as a potato self-incompatibility determining gene; the target fragment in the step (1) is located on the target gene S-RNase, and one strand of the target fragment has the nucleic acid sequence structure as shown in SEQ ID No: 1. One strand of the target fragment has the nucleic acid sequence structure as shown in SEQ ID No:1. For example, wherein the target fragment is located on the target S-RNase gene, one strand of the target fragment has a 5'-(N)_{X}-NGG-3' structure, and (N)_{X} represents a base sequence having the base number of X {N₁, N₂, ..., N_{X}}, and each of N₁, N₂, ... N_{X} represents any one of bases A, G, C, and T, and N in NGG is any one of A, G, C, and T.
(2) constructing a CRISPR/Cas9 recombinant vector for diploid potato S-RNase gene-targeting according to the nucleic acid sequence of the target fragment obtained in step (1), wherein in the step (2) the recombinant vector comprises the target fragment, wherein the target fragment is the nucleic acid sequence of SEQ ID No:1 or a sequence complementary thereof;
   The specific operations are as follows:
   2.1 selecting a completely conservative nucleotide sequence ACGATTCACGGGCTTTGGCCGG on the first exon of the two S-RNase genes (Sₚ₃ and Sₚ₄) of the diploid potato *S. phureja* CIP 703541 (the last CGG part is the NGG portion in the 5'-(N)_{X}-NGG-3' structure) as a targeting site. The nucleotide sequence of Sₚ₃ is shown as Seq ID No:2, the nucleotide sequence of Sₚ₄ is shown as Seq ID No:3; the target nucleotide sequence of Sₚ₃ is shown as positions 154 to 172 of Seq ID No:2, and the target nucleotide sequence of Sₚ₄ is shown as positions 157 to 175 of Seq ID No:3;
   2.2 synthesizing the forward oligonucleotide strand (S-RNase P3) and the complementary reverse oligonucleotide strand (S-RNase P4) according to the selected target site.
      The nucleotide sequence in the CRISPR/Cas9 recombinant vector comprises:
      S-RNase P3 (i.e., Seq ID No:4): xxxxACGATTCACGGGCTTTGGC,
      S-RNase P4 (i.e., Seq ID No:5): xxxxGCCAAAGCCCGTGAATCGT;
      wherein the portion not underlined is a sequence in above target site with deletion of NGG or a complementary sequence thereof, and the underlined portion is a cohesive end for ligation of the vector;
   2.3 annealing the primers S-RNase P3 and S-RNase P4, and the two strands of S-RNase P3 and S-RNase P4 are annealed to form double-stranded DNA with cohesive ends as an insert fragment for constructing a recombinant vector;
   2.4 digesting the pKSE401 vector with BsaI endonuclease at 50 °C for 12 hours, and inactivating the enzyme digestion system at 65 °C for 10 min, as a backbone fragment of the framework recombinant vector;
   2.5 ligating the recombinant vector backbone fragment and the insert fragment by T4 ligase, then transferring into *E. coli,* after verification by sequencing, the positive transformants are extracted to form a recombinant vector plasmid for targeting the diploid potato S-RNase gene by CRISPR/Cas9;
   2.6 transferring the recombinant vector plasmid into Agrobacterium EHA105 strain, and after sequencing, then extracting the positive transformed strain after verification by sequencing.
(3) introducing the recombinant vector obtained in the step (2) into potato cells, inducing the co-expression of the guide RNA expression cassette and the Cas9 nuclease expression cassette of the target fragment in the cell, cleaving the double-stranded target fragment of the S-RNase gene to trigger the DNA repair function of the potato cell itself, and causing random insertion or deletion of bases at the target site, thereby achieving a loss-of-function mutation of the intracellular S-RNase gene;
(4) regenerating a plurality of potato plants from the potato cells introduced with the recombinant vector, and screening the marker gene in the selected regeneration plants;
(5) specifically amplifying a DNA segment with the target fragment in the S-RNase gene of the selected regeneration plants by genomic PCR method, and sequencing the amplified products;
(6) selecting a regenerated plant in which the S-RNase gene is edited;
(7) detecting the ploidy of the selected gene-edited plant to select a diploid gene-edited plant line;
(8) propagating and planting the selected gene-edited plant line, and identifying the self-compatible phenotype at the flowering stage; and
(9) harvesting the seeds of the self-compatible plant line, extracting the genomic DNA of the offspring, and specifically amplifying a DNA segment with the target fragment in the S-RNase gene of the selected offspring by PCR method, then sequencing the amplified products and detecting the inheritance and isolation of the edited target gene in the offspring.

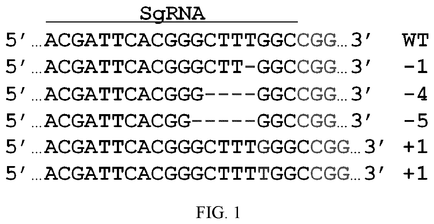

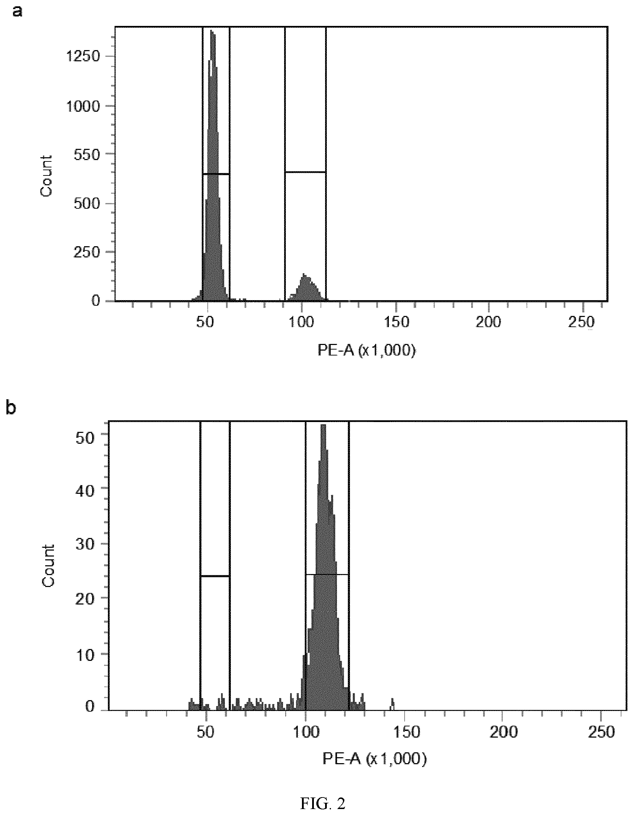

The specific operations for detecting the gene editing of the potato S-RNase in the above steps:
3.1 culturing the shoot tip of the aseptically preserved donor potato material *S. phureja* CIP 703541 on MS30 basal medium for 3 weeks, and taking the internodes as explants to plate on P-MS20 plate medium (2 pieces of sterile filter paper are previously placed on the surface of the medium, adding 2 mL of PACM solution) and pre-culturing for 2 days, the basic medium formula is as described in MS30, the pre-medium formula is as described in P-MS20, and the PACM solution is formulated as PACM;
3.2 activating positive transformed strain of Agrobacterium EHA105, shaking the bacteria to OD 0.5, then dipping and dyeing the pre-cultured explants described in the above step 2.1 for 15 minutes, then plating the explants on C-MS20 plate medium (1 piece of sterile filter paper is previously placed on the surface of the medium) and co-culturing in the dark for 2 days, and the common medium formulation is as described in C-MS20;
3.3 transferring the co-cultured explants from the end of step 3.2 onto D-MS20 plate differentiation medium for culturing, and the medium is changed every 14 days, and the differentiation medium formula is as described in D-MS20;
3.4 excising the extensible shoots produced by differentiation on the explants, and transferring onto the R-MS30 medium in the tissue culture flask for the resistance screening of the positive transformant, and the resistant screening medium formula is as described in R-MS30;
3.5 extracting the genomic DNA of the positive transformant as a template, and amplifying the full length of the two S-RNase genes Sₚ₃ and Sₚ₄ by respectively using the specific primer pairs, Sₚ₃-F: GGGGAAACTGGAAAATGGTT (i.e., Seq ID No:6), Sₚ₃-R: ATGTGAAGTTGTTCAGCGAAA (i.e., Seq ID No:7), and Sₚ₄-F: CAACAAAATGGCTAAATCGCAG (i.e., Seq ID No:8), Sₚ₄-R: GGTTTTCTGTTGGGTGGCAT (i.e., Seq ID No:9); then detecting the target mutation of the target gene sequence by Sanger sequencing, and the results are shown in Fig. 1;
   It can be seen from Fig. 1 that, in the present example five plants with target mutations are obtained, and all the S-RNase proteins undergo frameshift mutation.
3.6 detecting the positive transformants of the above target mutations by flow cytometry, and selecting the potato S-RNase gene-edited material, which still retains the diploid chromosomes. The detection results are shown in Fig. 2, wherein panel a is a diploid; panel b is a tetraploid. In the present example, five plants with target mutations are obtained, and their ploidy traits are all diploid types as shown in panel a.

### 4. Phenotypic identification of diploid potato S-RNase gene-edited material :

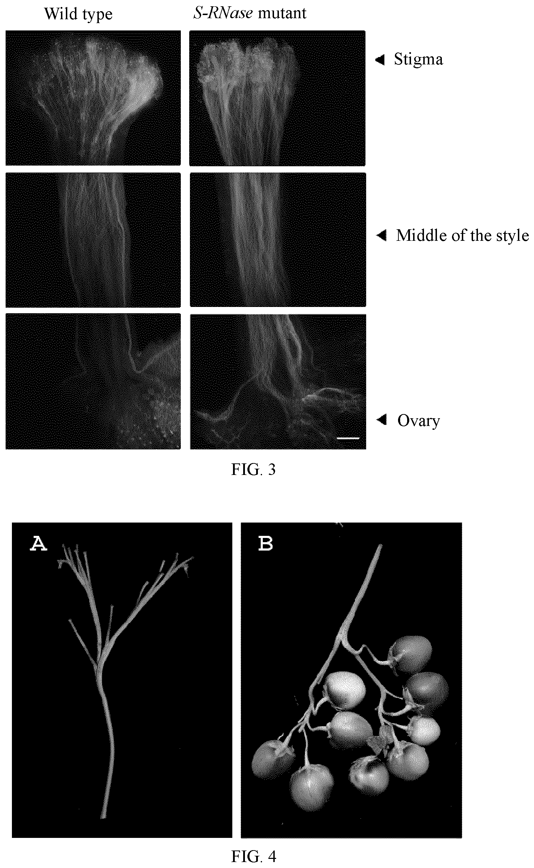

4.1 propagating and planting the diploid potato S-RNase gene-edited plant line, performing artificial self-crossing at the flowering stage;
4.2 48 hours after pollination, taking the pistil tissues of wild type and mutant plant lines respectively, fixing with 95% EtOH and glacial acetic acid in proportion of 3:1 for 24 hours, softening by 5M NaOH for 24 hours, and rinsing with ddH₂O, staining with 0.005 mg·mL⁻¹ aniline blue solution for 24 hours, and examining pollen tube dyeing under a fluorescence microscope. The detection results are shown in Fig. 3. According to the detection results, 48 hours after self-pollination, as for a group of wild-type materials *S.phureja* CIP 703541, the pollen tubes cannot enter the ovules, i.e., the wild type materials are self-incompatible; and 48 hours after self-pollination, as for the other group of donor material with orthomutation (S-RNase mutant), the pollen tubes successfully enter the ovules, indicating that the mutant plant line is self-compatible;
4.3 identifying the fruit setting phenotype of self-pollination for the wild-type and the mutant plant lines, and the detection results are shown in Fig. 4. Fig. 4A is a wild-type plant line, and Fig. 4B is a genetically edited plant line. It can be seen from Fig. 4 that, the wild type cannot bear fruits by self-crossing, and the mutant type can bear fruits by self-crossing, indicating that the mutant plant line is a self-compatible new material, and successfully overcomes the barrier of self-incompatibility;
4.4 harvesting the seeds of the self-compatible plant line, and sowing the seeds in the aperture disk; after the true leaves come out, extracting the genomic DNA of all the seedlings; then specifically amplifying the two DNA segments containing the target fragments in the S-RNase gene of the selected seedlings by PCR, and sequencing the amplified products to detect the inheritance and isolation of the edited target gene in the offspring. It is verified that the self-compatibility of the new material created by site-directed gene editing of the S-RNase can be passed on to the offspring. The verification results are shown in Table 1.

**Table 1: Mutation patterns of T0 and T1 generation plant lines of the gene-edited materials**

| No. | | T₀ generation | | T₁ generation | | |
|---|---|---|---|---|---|---|
| | *Sp3* | *Sp4* | Cas9-free^{a} | *Sp3Sp3*^{b} | *Sp3Sp4* | *Sp4Sp4* |
| 32 | chimeric | +1 bp | 7/192 | 0 | *3* (*Sp4*)^{c} | *4 (Sp4)* |
| 42 | wild type | -5 bp | 45/192 | 0 | *17 (Sp4)* | *28 (Sp4)* |
| 44 | -4 bp | chimeric | 47/192 | *20 (Sp3)* | *27 (Sp3)* | 0 |
| 57 | wild type | chimeric | 13/136 | 0 | *6 (Sp4)* | *7 (Sp4)* |
| 66 | -1 bp | wild type | 27/192 | *14 (Sp3)* | *13 (Sp3)* | 0 |

| | | | | | | |
|---|---|---|---|---|---|---|
| *Note:* ^{a} The number before the slash represents the number of individual plants without Cas9 in the detected T₁ generation, the number after the slash represents the number of individual plants in the detected T₁ generation; ^{b} the isolation of S-RNase type for the individual plants without Cas9 in the T₁ generation; ^{c} indicates the S-RNase mutation type. | | | | | | |

The medium formulations used above are shown in the following tables:
MS30 (1L):

| | |
|---|---|
| MS | 4.43 g |
| sucrose | 30 g |
| pH | 5.8 |
| agar | 8 g |

P-MS20 (1L):

| | |
|---|---|
| MS | 4.43 g |
| sucrose | 20 g |
| pH | 5.8 |
| agar | 8 g |

PA-MS20 (1L):

| | |
|---|---|
| MS | 4.43 g |
| sucrose | 20 g |
| caseine hydrolysate | 2 g |
| 2,4-D | 1 mg/L |
| KT | 0.5 mg/L |
| pH | 6.5 |

C-MS20 (1L):

| | |
|---|---|
| MS | 4.43 g |
| sucrose | 20 g |
| pH | 5.8 |
| agar | 8 g |
| a-napthaleneacetic acid | 2 mg·L⁻¹ |
| trans-zeatin | 1 mg·L⁻¹ |
| AS | 40 mg·L⁻¹ |

D-MS20 (1L):

| | |
|---|---|
| MS | 4.43 g |
| sucrose | 20 g |
| pH | 5.8 |
| agar | 8 g |
| a-napthaleneacetic acid | 0.01 mg·L⁻¹ |
| trans-zeatin | 2 mg·L⁻¹ |
| kanamycin | 100 mg·L⁻¹ |
| temetine | 200 mg·L⁻¹ |

R-MS30 (1L):

| | |
|---|---|
| MS | 4.43 g |
| sucrose | 30 g |
| pH | 5.8 |
| agar | 8 g |
| kanamycin | 50 mg·L⁻¹ |
| temetine | 200 mg·L⁻¹ |

## Claims

1. A CRISPR/Cas9 recombinant vector for targeted knockout of S-RNase protein genes, **characterized in that** the nucleotide sequence of the S-RNase protein targeted by the CRISPR/Cas9 recombinant vector is the sequence shown in SEQ ID NO:2 (Sₚ₃), or a complementary sequence, a degenerate sequence, or a homologous sequence thereof; and/or the sequence shown in SEQ ID NO:3 (Sₚ₄), or a complement sequence, a degenerate sequence, or a homologous sequence thereof; wherein the nucleotide sequence in the CRISPR/Cas9 recombinant vector comprises:
S-RNase P3 (i.e., Seq ID No:4): xxxxACGATTCACGGGCTTTGGC, and
S-RNase P4 (i.e., Seq ID No:5): xxxxGCCAAAGCCCGTGAATCGT;
wherein, in Seq ID No.4 and Seq ID No.5, xxxx is a cohesive end for ligation of the vector.

2. Use of the CRISPR/Cas9 recombinant vector of claim 1 for knocking out an S-RNase protein gene of the diploid potato S.phureja.

3. A method for creating a self-compatible potato, **characterized by** consisting of:
(1) constructing a CRISPR/Cas9 recombinant vector of claim 1;
(2) introducing by transformation the CRISPR/Cas9 recombinant vector in step (1) into potato cells, comprising the co-expression of the guide RNA expression cassette and the Cas9 nuclease expression cassette of the target fragment in the cell, cleaving the double-stranded target fragment of the S-RNase gene to trigger the DNA repair function of the potato cell itself, and causing random insertion or deletion of bases at the target site, thereby achieving a loss-of-function mutation of the intracellular S-RNase gene;
(3) screening for plants with a mutation in the S-RNase gene;
wherein the S-RNase gene is the sequence shown in SEQ ID NO:2 (Sₚ₃), or a complementary sequence, a degenerate sequence, or a homologous sequence thereof; and/or the sequence shown in SEQ ID NO:3 (Sₚ₄), or a complement sequence, a degenerate sequence, or a homologous sequence thereof; and the potato is S.phureja.

## Patentansprüche

1. Ein rekombinanter CRISPR/Cas9-Vektor für den zielgerichteten Knockout von S-RNase-Proteingenen, **dadurch gekennzeichnet, dass** die Nukleotidsequenz des S-RNase-Proteins, auf das der rekombinante CRISPR/Cas9-Vektor abgezielt ist, die in SEQ ID NO: 2 (Sₚ₃) gezeigte Sequenz oder eine komplementäre Sequenz, eine degenerierte Sequenz oder eine homologe Sequenz davon ist; und/oder die in SEQ ID NO: 3 (Sₚ₄) gezeigte Sequenz oder eine komplementäre Sequenz, eine degenerierte Sequenz oder eine homologe Sequenz davon ist; wobei die Nukleotidsequenz in dem rekombinanten CRISPR/Cas9-Vektor Folgendes beinhaltet:
S-RNase P3 (d. h. SEQ ID NO: 4): xxxxACGATTCACGGGCTTTGGC, und
S-RNase P4 (d.h. SEQ ID NO: 5) xxxxGCCAAAGCCCGTGAATCGT;
wobei xxxx in SEQ ID NO: 4 und SEQ ID NO: 5 ein kohäsives Ende für die Ligation des Vektors ist.

2. Verwendung des rekombinanten CRISPR/Cas9-Vektors gemäß Anspruch 1 für den Knockout eines S-RNase-Proteingens der diploiden Kartoffel S. phureja.

3. Ein Verfahren zum Erzeugen einer selbstkompatiblen Kartoffel, **dadurch gekennzeichnet, dass** es aus Folgendem besteht:
(1) Konstruieren eines rekombinanten CRISPR/Cas9-Vektors gemäß Anspruch 1;
(2) Einführen des rekombinanten CRISPR/Cas9-Vektors von Schritt (1) in Kartoffelzellen durch Transformation, beinhaltend die Coexpression der Guide-RNA-Expressionskassette und der Cas9-Nuklease-Expressionskassette des Zielfragments in der Zelle, Spalten des doppelsträngigen Zielfragments des S-RNase-Gens, um die eigene DNA-Reparaturfunktion der Kartoffelzelle auszulösen, und Bewirken einer zufälligen Insertion oder Deletion von Basen an der Zielstelle, dadurch Erreichen einer Funktionsverlustmutation des intrazellulären S-RNase-Gens;
(3) Durchführen eines Screenings auf Pflanzen mit einer Mutation in dem S-RNase-Gen;
wobei das S-RNase-Gen die in SEQ ID NO: 2 (Sₚ₃) gezeigte Sequenz oder eine komplementäre Sequenz, eine degenerierte Sequenz oder eine homologe Sequenz davon ist; und/oder die in SEQ ID NO: 3 (Sₚ₄) gezeigte Sequenz oder eine komplementäre Sequenz, eine degenerierte Sequenz oder eine homologe Sequenz davon ist; und die Kartoffel S. phureja ist.

## Revendications

1. Un vecteur recombinant CRISPR/Cas9 pour l'invalidation ciblée de gènes de protéine S-RNase, **caractérisé en ce que** la séquence nucléotidique de la protéine S-RNase ciblée par le vecteur recombinant CRISPR/Cas9 est la séquence montrée dans SEQ ID NO : 2 (Sₚ₃), ou une séquence complémentaire, une séquence dégénérée, ou une séquence homologue de celle-ci ; et/ou la séquence montrée dans SEQ ID NO : 3 (Sₚ₄), ou une séquence complémentaire, une séquence dégénérée, ou une séquence homologue de celle-ci ; où la séquence nucléotidique dans le vecteur recombinant CRISPR/Cas9 comprend :
S-RNase P3 (c.-à-d., Seq ID N° : 4) : xxxxACGATTCACGGGCTTTGGC, et
S-RNase P4 (c.-à-d., Seq ID N° : 5) : xxxxGCCAAAGCCCGTGAATCGT ;
où, dans Seq ID N° 4 et Seq ID N° 5, xxxx est une extrémité cohésive pour la ligature du vecteur.

2. Utilisation du vecteur recombinant CRISPR/Cas9 de la revendication 1 pour invalider un gène de protéine S-RNase de la pomme de terre diploïde S.phureja.

3. Un procédé pour créer une pomme de terre auto-compatible, **caractérisé en ce qu'**il consiste en :
(1) le fait de construire un vecteur recombinant CRISPR/Cas9 de la revendication 1 ;
(2) le fait d'introduire par transformation le vecteur recombinant CRISPR/Cas9 de l'étape (1) dans des cellules de pomme de terre, comprenant la co-expression de la cassette d'expression de l'ARN guide et de la cassette d'expression de la nucléase Cas9 du fragment cible dans la cellule, le fait de cliver le fragment cible double brin du gène de S-RNase afin de déclencher la fonction de réparation de l'ADN de la pomme de terre elle-même, et le fait de causer l'insertion ou la délétion aléatoires de bases au niveau du site cible, obtenant par là une mutation de perte de fonction du gène de S-RNase intracellulaire ;
(3) le fait d'effectuer un criblage à la recherche de plantes possédant une mutation du gène de S-RNase ;
où le gène de S-RNase est la séquence montrée dans SEQ ID NO : 2 (Sₚ₃), ou une séquence complémentaire, une séquence dégénérée, ou une séquence homologue de celle-ci ; et/ou la séquence montrée dans SEQ ID NO : 3 (Sₚ₄), ou une séquence complémentaire, une séquence dégénérée, ou une séquence homologue de celle-ci ; et la pomme de terre est S.phureja.
